# EUROPÄISCHE PATENTSCHRIFT

(11) **EP 0 768 879 B1**
(45) Veröffentlichungstag und Bekanntmachung des Hinweises auf die Patenterteilung: **19.03.2003**
(21) Anmeldenummer: 95926897.0
(22) Anmeldetag: 14.07.1995
(51) Int. Cl.: A61K 31/435, A61K 31/44, A61K 31/445

(54) **THIADIAZOLVERBINDUNGEN ALS D3 DOPAMINREZEPTORLIGAND**
THIADIAZOLE COMPOUNDS AS D3 DOPAMINE RECEPTOR LIGANDS
COMPOSES DE THIADIAZOLE COMME LIGANDS DU RECEPTEUR DE LA DOPAMINE D3

(30) Priorität: 15.07.1994 DE 4425145
(43) Veröffentlichungstag der Anmeldung: 23.04.1997
(73) Patentinhaber: BASF AKTIENGESELLSCHAFT, 67056 Ludwigshafen (DE)
(72) Erfinder: HELLENDAHL, Beate, 67105 Schifferstadt (DE); LANSKY, Annegret, 64297 Darmstadt (DE); RENDENBACH-MÜLLER, Beatrice, 67435 Neustadt (DE); BACH, Alfred, 69123 Heidelberg (DE); UNGER, Liliane, 67065 Ludwigshafen (DE); TESCHENDORF, Hans-Jürgen, 67373 Dudenhofen (DE); WICKE, Karsten, 67122 Altrip (DE)
(74) Vertreter: Kinzebach, Werner, Dr.
(86) Internationale Anmeldenummer: EP9502783
(87) Internationale Veröffentlichungsnummer: WO96002249

(56) Entgegenhaltungen:
- EP-A- 0 282 133
- EP-A- 0 345 533
- EP-A- 0 356 333
- EP-A- 0 409 048
- WO-A-92/07831
- WO-A-92/22540
- WO-A-92/22541
- WO-A-92/22542
- GB-A- 1 149 110
- THE JOURNAL OF MEDICINAL CHEMISTRY, Bd. 34, Nr. 6, 1991 Seiten 1860-1866, LOWE, J.A. ET AL '1-Naphthylpiperazine derivatives as potential atypical antipsychotic agents'

## Beschreibung

Die Erfindung betrifft Thiadiazolverbindungen. Die erwähnten Verbindungen besitzen wertvolle therapeutische Eigenschaften und sind zur Behandlung von Erkrankungen brauchbar, die auf Dopamin-D₃-Rezeptorliganden ansprechen.

Verbindungen der hier in Rede stehenden Art mit physiologischer Aktivität sind bereits bekannt. So beschreibt die US-A-4,074,049 Aminoalkylthiothiadiazole, die fungizide und Blutplättchen-aggregationshemmende Wirkung aufweisen. Die JP-A-2 153 276 beschreibt ähnliche Verbindungen, die zur Behandlung von Erkrankungen der Leber Verwendung finden.

In der GB-A-1 053 085 werden Aminoalkylthiadiazole beschrieben, die antitussive, analgetische, antipyretische und hypoglykämische Wirkungen zeigen. In der US-A-3,717,651 sind 5-Mercapto-substituierte Thiazole beschrieben, welche herbizide Eigenschaften besitzen.

Die WO 89/11 476 beschreibt substituierte 2-Aminothiazole als dopaminerge Mittel, die unter anderem zur Behandlung von Psychosen und Erkrankungen des ZNS brauchbar sind.

Die WO 92/22 540 beschreibt aminoalkylsubstituierte 5-Mercaptothiazole der Formel: 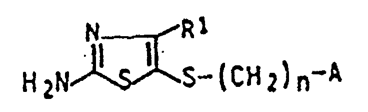 worin R¹ für H, C₁-C₅-Alkyl, gegebenenfalls substituiertes Phenyl oder Thienyl steht; n für 2 bis 6 steht; und A für 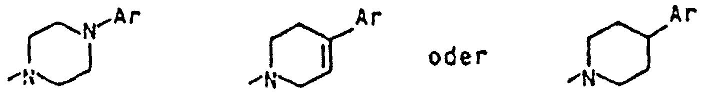 steht, wobei Ar für einen gegebenenfalls einfach durch C₁-C₅-Alkyl, C₁-C₅-Alkoxy, Amino, Halogen, Nitro, Hydroxy, Trifluormethyl oder Cyano substituierten Phenylring, einen Pyridyl-, Pyrimidinyl- oder Thienylrest steht. Diese Verbindungen sind zur Behandlung von Erkrankungen des zentralen Nervensystems, z.B. Parkinsonismus, Schizophrenie, und von Erkrankungen, die mit erhöhtem Blutdruck einhergehen, brauchbar.

Die WO 92/22 542 beschreibt die entsprechenden 2-Amino-5-mercapto-1,3,4-thiadiazolderivate, die ebenfalls zur Behandlung von Erkrankungen des zentralen Nervensystems und von Erkrankungen, die mit erhöhtem Blutdruck einhergehen, brauchbar sind.

Die WO 92/22 541 beschreibt entsprechende 2-Amino-1,3,4-thiadiazolderivate, wobei die Alkylenkette nicht über ein Schwefelatom, sondern direkt an den Thiadiazolrest gebunden ist. Auch diese Verbindungen sind zur Behandlung von Erkrankungen des zentralen Nervensystems und von Erkrankungen, die mit erhöhtem Blutdruck einhergehen, brauchbar.

Die WO 93/21 179 beschreibt 1-Aryl-4-(ω-amido-1-alkyl und ω-imido-1-alkyl)piperazin-Verbindungen. Diese Verbindungen stellen Dopamin-D₂-Rezeptorantagonisten und 5-HT_{1A}-Rezeptoragonisten dar. Sie sind als antipsychotische Mittel, beispielsweise zur Behandlung von Schizophrenie, brauchbar.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin.

Es liegen gesicherte Erkenntnisse über die Anwesenheit von Dopamin und dessen physiologische Funktion als Neurotransmitter vor. Auf Dopamin ansprechende Zellen stehen im Zusammenhang mit der Etiologie von Schizophrenie und der Parkinson' schen Krankheit. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptor pharmakologisch klar definiert, nämlich die D₁- und D₂-Rezeptoren.

Sokoloff et al., Nature 1990, 347:146-151, hat einen dritten Subtyp gefunden, nämlich die D₃-Rezeptoren. Sie werden hauptsächlich im limbischen System exprimiert. Strukturell unterscheiden sich die D₃-Rezeptoren von den D₁- und D₂₋ Rezeptoren in etwa der Hälfte der Aminosäurereste.

Die Wirkung von Neuroleptika wurde im allgemeinen ihrer Affinität zu den D₂-Rezeptoren zugeschrieben. Neuere Rezeptorbindungsstudien haben dies bestätigt. Danach besitzen die meisten Dopaminantagonisten, wie Neuroleptika, hohe Affinität zu den D₂₋ Rezeptoren, aber nur geringe Affinität zu den D₃-Rezeptoren.

Bei den oben beschriebenen Verbindungen des Standes der Technik handelt es sich um solche D₂-Rezeptoragonisten bzw. -antagonisten.

Überraschenderweise wurde nun gefunden, daß die erfindungsgemäßen Verbindungen eine hohe Affinität zum Dopamin-D₃-Rezeptor und nur geringe Affinität zum D₂-Rezeptor aufweisen. Es handelt sich somit um selektive D₃-Liganden.

Gegenstand der vorliegenden Erfindung sind Thiadiazolverbindungen der Formel I: 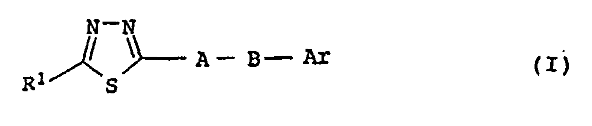 worin
- A: für eine geradkettige oder verzweigte C₃-C₁₄-Alkylengruppe steht, die gegebenenfalls wenigstens eine Gruppe umfasst, die ausgewählt ist unter O, S, NR³ und einer Doppel- oder Dreifachbindung,
- B: für einen Rest der Formel steht: 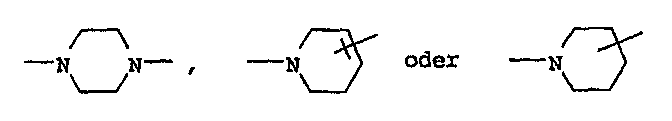
- R¹: für Wasserstoff, OR³, NR²R³ oder für C₁-C₈-Alkyl steht,
- R² und R³: unabhängig voneinander für H oder C₁-C₈-Alkyl stehen,
- Ar: für Pyridinyl, Pyrimidinyl oder Triazinyl steht, wobei Ar ein bis vier Substituenten aufweist, die ausgewählt sind unter OR³, C₁-C₈-Alkyl, C₂-C₈-Alkinyl, C₂-C₈-Alkenyl, Halogen, CN, CO₂R², NO₂, SO₂R², SO₃R², NR²R³, SO₂NR²R³, SR², CHF₂, CF₃, einem 5- oder 6-gliedrigen, carbocyclischen, aromatischen oder nicht aromatischen Ring und einem 5- oder 6-gliedrigen, heterocyclischen, aromatischen oder nicht aromatischen Ring mit 1 bis 3 Heteroatomen, die ausgewählt sind unter O, S und N, wobei der carbocyclische oder der heterocyclische Ring gegebenenfalls substituiert ist durch C₁-C₈-Alkyl, OC₁-C₈-Alkyl, Halogen, OH, NO₂, oder CF₃, oder worin Ar mit einem carbocyclischen oder heterocyclischen Ring der oben genannten Art kondensiert ist,
sowie der Salze davon mit physiologisch verträglichen Säuren.

Bei den erfindungsgemäß zur Anwendung kommenden Verbindungen handelt es sich um selektive Dopamin-D₃-Rezeptor-Liganden, die regioselektiv im limbischen System angreifen und aufgrund ihrer geringen Affinität zum D₂-Rezeptor nebenwirkungsärmer als klassiche Neuroleptika sind. Die Verbindungen sind daher zur Behandlung von Erkrankungen brauchbar, die auf Dopamin-D₃-Rezeptorantagonisten bzw. -agonisten ansprechen, z.B. zur Behandlung von Erkrankungen des zentralen Nervensystems, insbesondere Schizophrenie, Depressionen, Neurosen und Psychosen. Außerdem sind sie zur Behandlung von Schlafstörungen und Übelkeit und als Antihistaminika brauchbar.

Im Rahmen der vorliegenden Erfindung besitzen die nachfolgenden Ausdrücke die anschließend angegebenen Bedeutungen:

Alkyl (auch in Resten wie Alkoxy, Alkylamino etc.) bedeutet eine geradkettige oder verzeigte Alkylgruppe mit 1 bis 8 Kohlenstoffatomen, vorzugsweise 1 bis 6 Kohlenstoffatomen und insbesondere 1 bis 4 Kohlenstoffatomen. Die Alkylgruppe kann einen oder mehrere Substituenten aufweisen, die unabhängig voneinander ausgewählt sind unter OH und OC₁-C₈-Alkyl.

Beispiele für eine Alkylgruppe sind Methyl, Ethyl, n-Propyl, i-Propyl, n-Butyl, iso-Butyl, t-Butyl etc.

Alkylen steht für geradkettige oder verzweigte Reste mit vorzugsweise 3 bis 12 Kohlenstoffatomen und insbesondere 7 bis 12 Kohlenstoffatomen.

Die Alkylengruppen können gegebenenfalls wenigstens eine der oben angegebenen Gruppen umfassen. Diese kann - ebenso wie die erwähnte Doppel- oder Dreifachbindung - in der Alkylenkette an beliebiger Stelle oder am Ende der Kette angeordnet sein so, daß sie die Kette mit dem Thiadiazolrest verbindet.

Halogen bedeutet F, Cl, Br, I und insbesondere F, Cl, Br.

Vorzugsweise steht R¹ für H, OR³ oder NR²R³, wobei R² und R³ unabhängig voneinander für H oder C₁-C₈-Alkyl stehen.

Der Rest Ar weist einen, zwei, drei oder vier Substituenten auf. Vorzugsweise sind sie unabhängig voneinander ausgewählt unter C₁-C₈-Alkyl, Phenyl, C₅-C₆-Cycloalkyl, einem 5- oder 6-gliedrigen heterocyclischen aromatischen oder nicht-aromatischen Rest mit 1 bis 3 Heteroatomen, ausgewählt unter O, N und S, CHF₂, CF₃, Halogen, NO₂, CN, OR³ und SR², wobei R² und R³ die oben angegebenen Bedeutungen besitzen.

Wenn einer der Substituenten des Restes Ar für C₁-C₈-Alkyl steht, ist ein verzweigter Rest, insbesondere die Isopropyl- oder t-Butylgruppe bevorzugt.

Ar weist mindestens einen Substituenten auf und steht insbesondere für 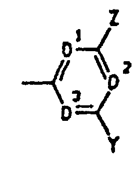 worin D¹, D² und D³ unabhängig voneinander für CR oder N stehen und R, Y und Z für H oder die oben bzw. nachfolgend angegebenen Bedeutungen stehen und wenigstens D¹, D² oder D³ für N steht.

Vorzugsweise steht Ar für substituiertes 2-, 3- oder 4-Pyridinyl oder 2-, 4(6)- oder 5-Pyrimidinyl.

Wenn einer der Substituenten des Restes Ar für einen 5- oder 6-gliedrigen heterocyclischen Ring steht, so handelt es sich beispielsweise um einen Pyrrolidin-, Piperidin-, Morpholin-, Piperazin-, Pyridin-, Pyrimidin-, Triazin-, Pyrrol-, Thiophen-, Thiazol-, Imidazol-, Oxazol-, Isoxazol-, Pyrazol- oder Thiadiazolrest.

Wenn einer der Substituenten des Restes Ar für einen carbocyclischen Rest steht, handelt es sich insbesondere um einen Phenyl-, Cyclopentyl- oder Cyclohexylrest.

Wenn Ar mit einem carbocyclischen oder heterocyclischen Rest kondensiert ist, steht Ar insbesondere für einen Chinolin-, Di- oder Tetrahydrochinolinrest.

Eine bevorzugte Ausführungsform sind die Verbindungen der Formel I, worin A für C₃-C₁₂-Alkylen steht, das gegebenenfalls wenigstens eine Gruppe umfaßt, die ausgewählt ist unter O, S, NR³ und einer Doppel- oder Dreifachbindung.

Eine weitere bevorzugte Ausführungsform sind die Verbindungen der Formel I, worin
- R¹: für H, OR³ oder NR²R³ steht, wobei R² und R³ unabhängig voneinander für H oder C₁-C₈-Alkyl stehen,
- A: für C₃-C₁₂-Alkylen steht, das gegebenenfalls wenigstens eine Gruppe umfaßt, die ausgewählt ist unter O, S, NR³ und einer Doppel- oder Dreifachbindung;
- Ar: für Pyrimidinyl oder Pyridyl steht, das einen, zwei, drei oder vier Substituenten aufweist, die unabhängig voneinander ausgewählt sind unter C₁-C₈-Alkyl, Phenyl, C₅-C₆-Cycloalkyl, einem 5- oder 6-gliedrigen heterocyclischen aromatischen oder nicht-aromatischen Rest mit 1 bis 3 Heteroatomen, ausgewählt unter O, N und S, CHF₂, CF₃, Halogen, NO₂, CN, OR³ und SR², wobei R² und R³ die oben angegebenen Bedeutungen besitzen.

Besonders bevorzugt sind dabei Verbindungen der Formel I, worin
- B: für 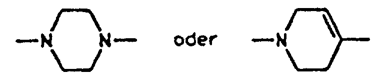 steht.

Eine weitere bevorzugte Ausführungsform sind die Verbindungen der Formel I, worin Ar für Pyrimidinyl steht, das ein bis drei Substituenten aufweist, die unabhängig voneinander ausgewählt sind unter, C₁-C₈-Alkyl, C₅-C₆-Cycloalkyl, Phenyl, Pyrrolyl, OH, OC₁-C₈-Alkyl, CHF₂, CF₃ und Halogen, oder worin Ar für Pyridinyl steht, das ein bis vier Substituenten aufweist, die unabhängig voneinander ausgewählt sind unter, C₁-C₈-Alkyl, C₂-C₈-Alkenyl, C₂-C₈-Alkinyl, Phenyl, Pyrrolyl, OH, OC₁-C₈-Alkyl, CHF₂, CF₃, CN und Halogen.

Die Erfindung umfaßt auch die Säureadditionssalze der Verbindungen der Formel I, mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff, Birkhäuser Verlag, Basel und Stuttgart, 1966, beschrieben.

Die Verbindungen der Formeln I können ein oder mehrere Asymmetrizentren aufweisen. Zur Erfindung zählen daher nicht nur die Racemate, sondern auch die betreffenden Enantiomere und Diastereomere. Auch die jeweiligen tautomeren Formen zählen zur Erfindung.

Die Herstellung der Verbindungen der Formeln I kann analog zu üblichen Methoden erfolgen, wie z. B. beschrieben in Houben Weyl, "Handbuch der Organischen Chemie", Ernst Schaumann (Hrsg.), 4. Aufl. Thieme Verlag, Stuttgart 1994, Band E8/d, S. 189ff.; A. R. Katritzky, C.W. Rees (ed.), "Comprehensive Heterocyclic Chemistry", 1. Aufl., und der dort zitierten Literatur. Das Verfahren zur Herstellung der Verbindungen besteht darin, daß man
i) eine Verbindung der allgemeinen Formel II: 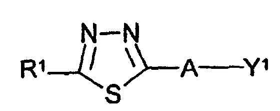 worin Y¹ für eine übliche Abgangsgruppe steht, mit einer Verbindung der allgemeinen Formel III:

   H - B - Ar

   umsetzt;
ii) zur Herstellung einer Verbindung der Formel I, worin A ein Sauerstoff- oder Schwefelatom oder NR³ umfaßt:
   a) einer Verbindung der allgemeinen Formel IV: 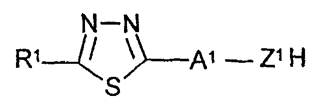
worin Z¹ für O,S oder NR³ steht und A¹ für C₀-C₁-Alkylen steht, mit einer Verbindung der allgemeinen Formel VI

Y¹ - A² - B - Ar

worin Y¹ die oben angegebenen Bedeutungen besitzt und A² für C₁-C₁₄-Alkylen steht, wobei A¹ und A² zusammen 7 bis 14 Kohlenstoffatome aufweisen, umsetzt;

Zur Behandlung der oben erwähnten Erkrankungen werden die erfindungsgemäßen Verbindungen in üblicher Weise oral oder parenteral (subkutan, intravenös, intramuskulär, intraperitoneal) verabreicht. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis etwa 10 bis 1000 mg pro Patient und Tag bei oraler Gabe und etwa 1 bis 500 mg pro Patient und Tag bei parenteraler Gabe.

Die Erfindung betrifft auch pharmazeutische Mittel, die die erfindungsgemäßen Verbindungen enthalten. Diese Mittel liegen in den üblichen galenischen Applikationsformen in fester oder flüssiger Form vor, beispielsweise als Tabletten, Filmtabletten, Kapseln, Pulver, Granulate, Dragees, Suppositorien, Lösungen oder Sprays. Die Wirkstoffe können dabei mit den üblichen galenischen Hilfsmitteln, wie Tablettenbindemitteln, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al., Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die nachfolgenden Beispiele dienen zur Erläuterung der Erfindung, ohne sie zu begrenzen.

### Referenz beispiel 1:

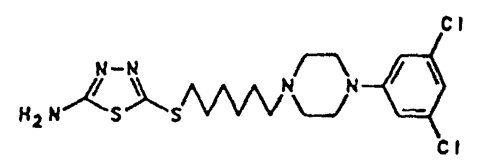

a) 2-Amino-5-(6-chlorhexylmercapto)-1,3,4-thiadiazol
   5 g 2-Amino-5-mercapto-1,3,4-thiadiazol, 7,5 g 1,6-Bromchlorhexan und 4,07 g Triethylamin wurden in 100 ml Tetrahydrofuran 1 Stunde unter Rückfluß erhitzt. Die Reaktionsmischung wurde abgesaugt, das Filtrat eingeengt, der verbleibende Rückstand mit Wasser gewaschen und anschließend getrocknet. Es verbleiben 8,2 g ( 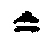 91 % Ausbeute) Produkt.
b) 2-Amino-5-(6-(3,5-dichlorphenylpiperazinyl)hexylmercapto)-1,3,4-thiadiazol)
   2,2 g Produkt aus 1a), 2,08 g 3,5-Dichlorphenylpiperazin und 1 g Triethylamin wurden in 4 ml DMF 1 Stunde auf 100° C erhitzt. Die Reaktionsmischung wurde mit Wasser versetzt, 3-mal mit Methylenchlorid extrahiert, über MgSO₄ getrocknet, eingeengt und der Rückstand chromatographisch gereinigt (Laufmittel: CH₂Cl/CH₃OH = 9/1). Man erhielt 1,0 g ( 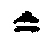 25 % Ausbeute) Produkt. Smp.: 130° C.

### Referenz beispiel 2:

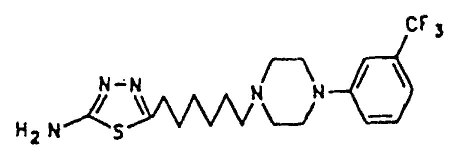

a) 5-Amino-2-(6-bromhexyl)-1,3,4-thiadiazol
   5 g 7-Bromheptansäure und 2,17 g Thiosemicarbazid wurden in 50 ml Dioxan bei 90° C vorgelegt und bei dieser Temperatur 4,0 g POCl₃ zugetropft. Anschließend wurde 1 Stunde bei 90°C gerührt. Dann wurde die Reaktionsmischung mit Wasser versetzt und 3-mal mit Methylenchlorid extrahiert, über MgSO₄ getrocknet und eingeengt. Man erhielt 6,1 g ( 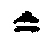 96 % Ausbeute) Produkt als Rückstand.
b) 2,5 g Produkt 2a), 2,18 g m-Trifluormethylphenylpiperazin und 1,92 g Triethylamin wurden in 3 ml DMF 1 Stunde bei 100° C gerührt. Die Aufarbeitung erfolgte wie unter 1b). Man erhielt 1,4 g ( 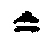 36 % Ausbeute) Produkt. Smp.: 134-136° C.

Die in den nachfolgenden Tabellen 1 bis 5 aufgeführten Verbindungen können ebenfalls in analoger Weise hergestellt werden.

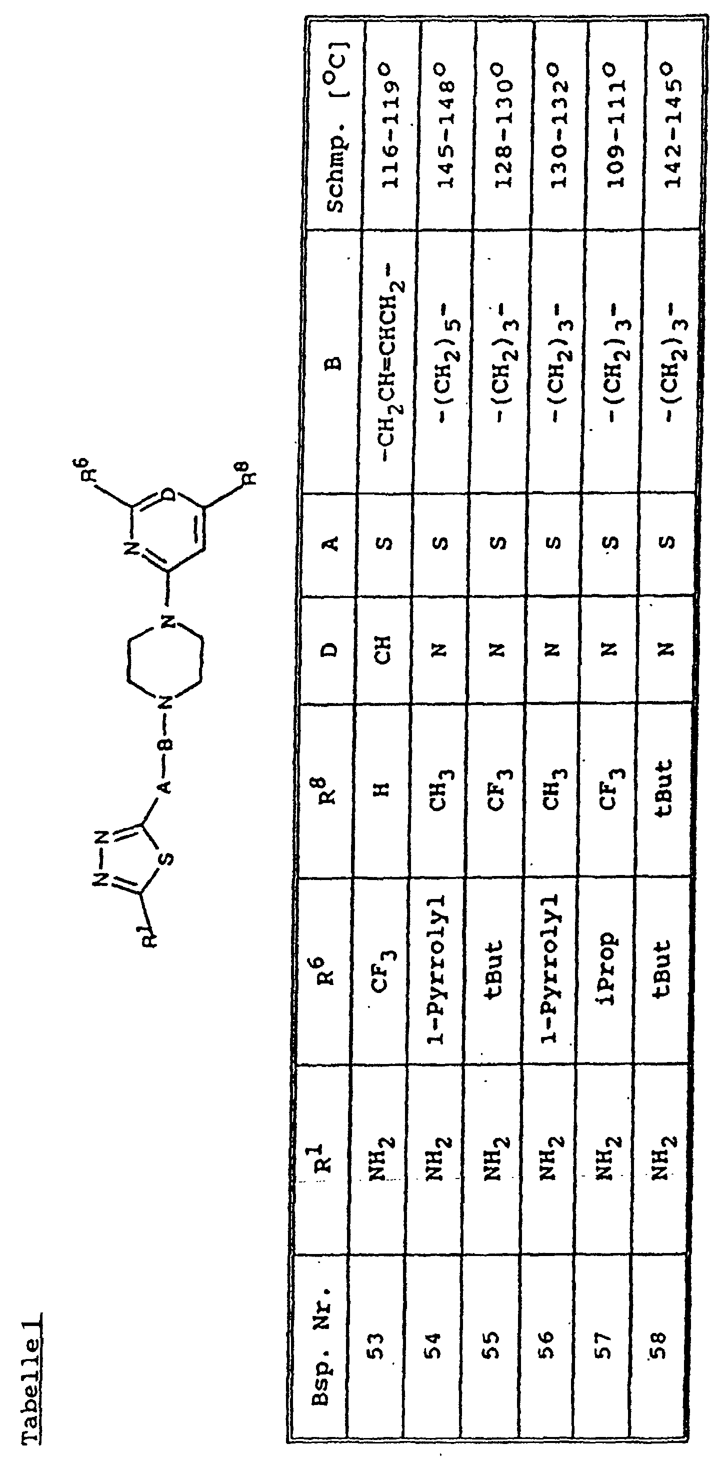

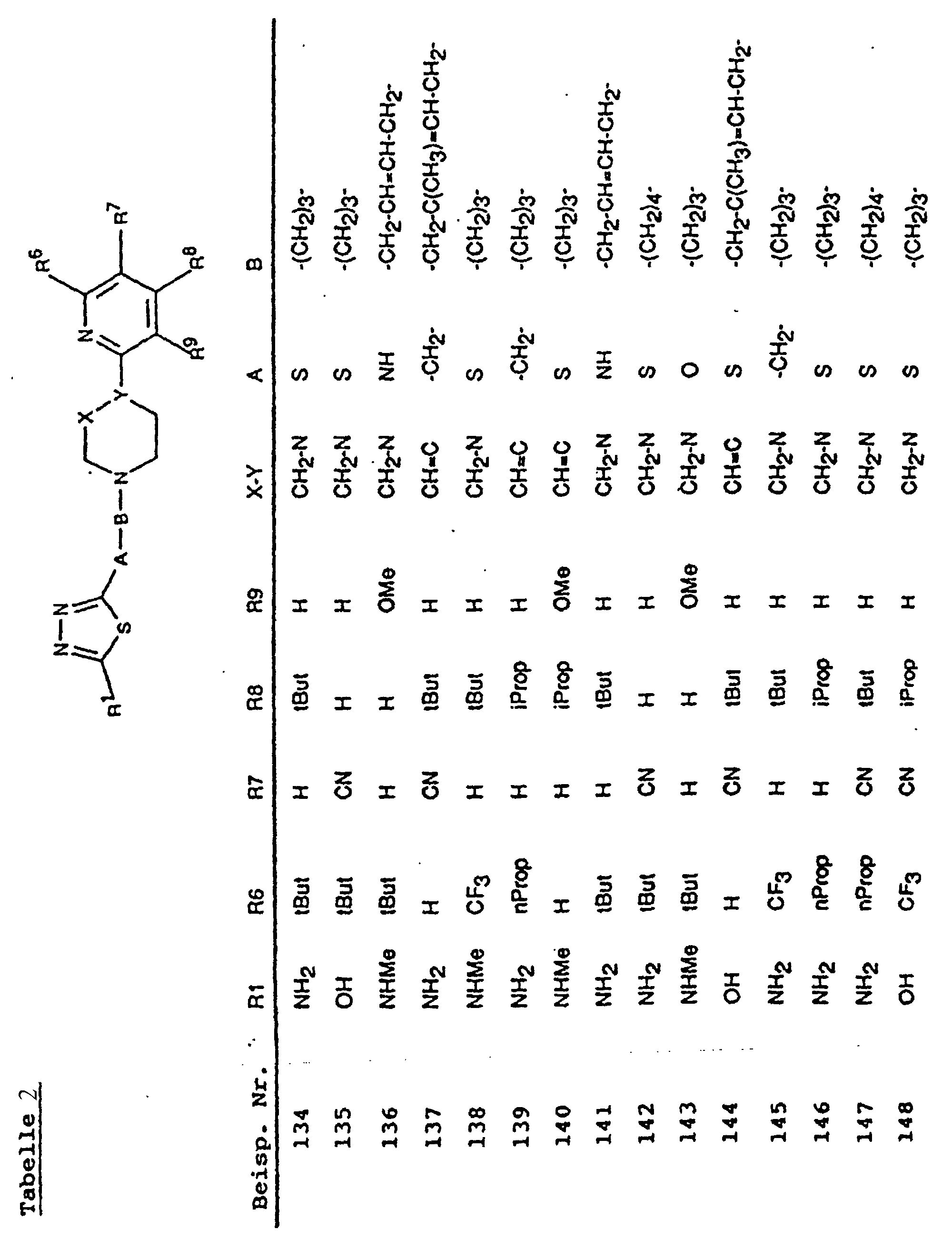

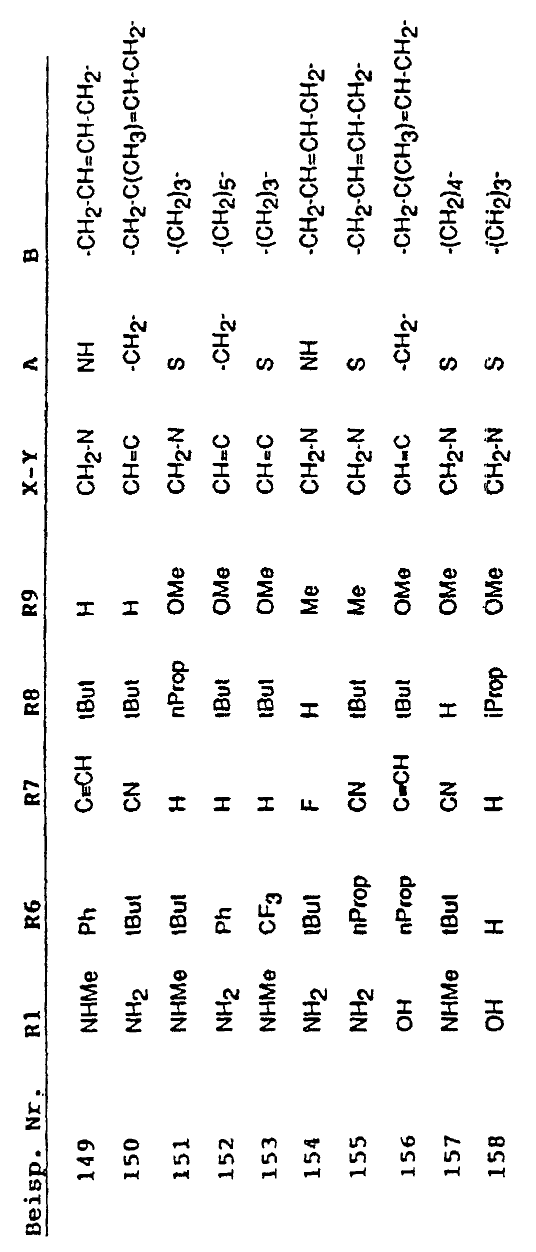

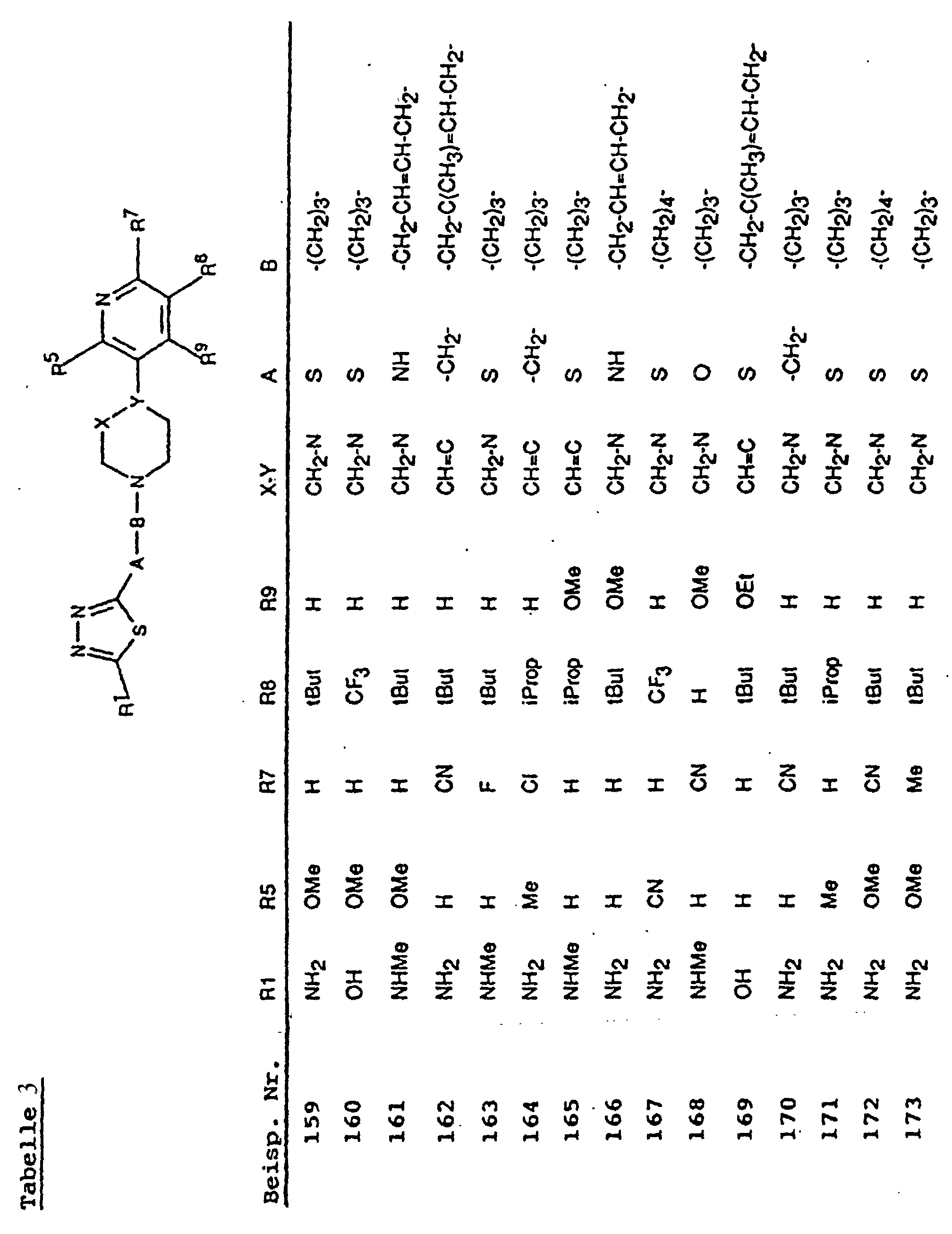

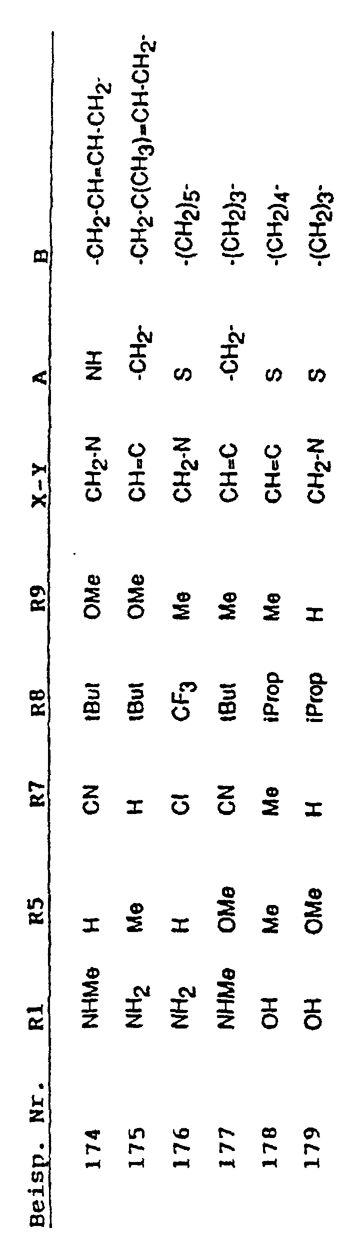

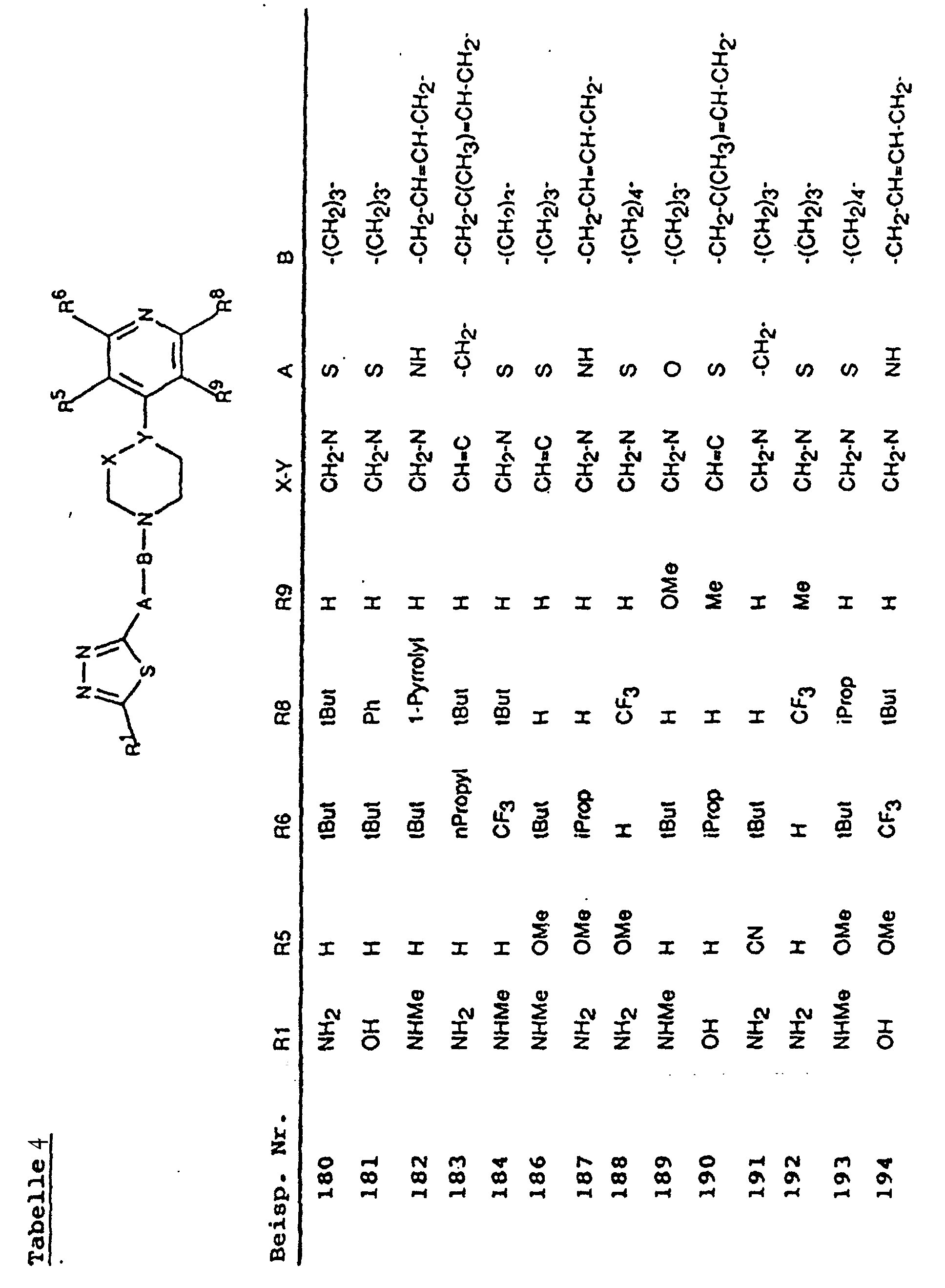

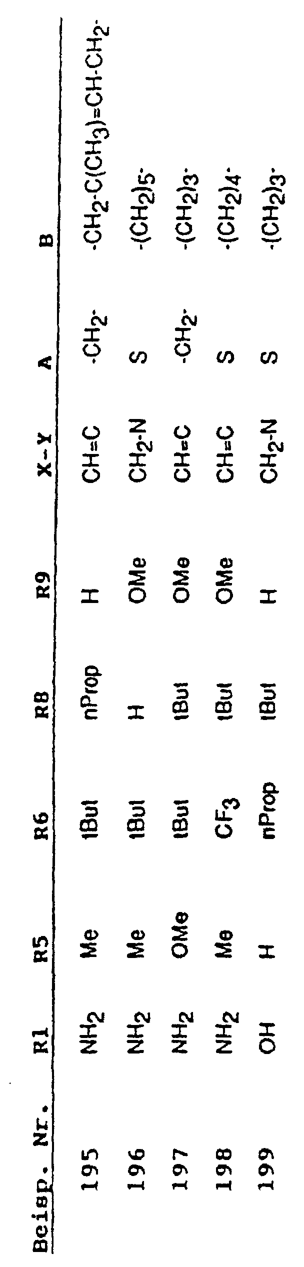

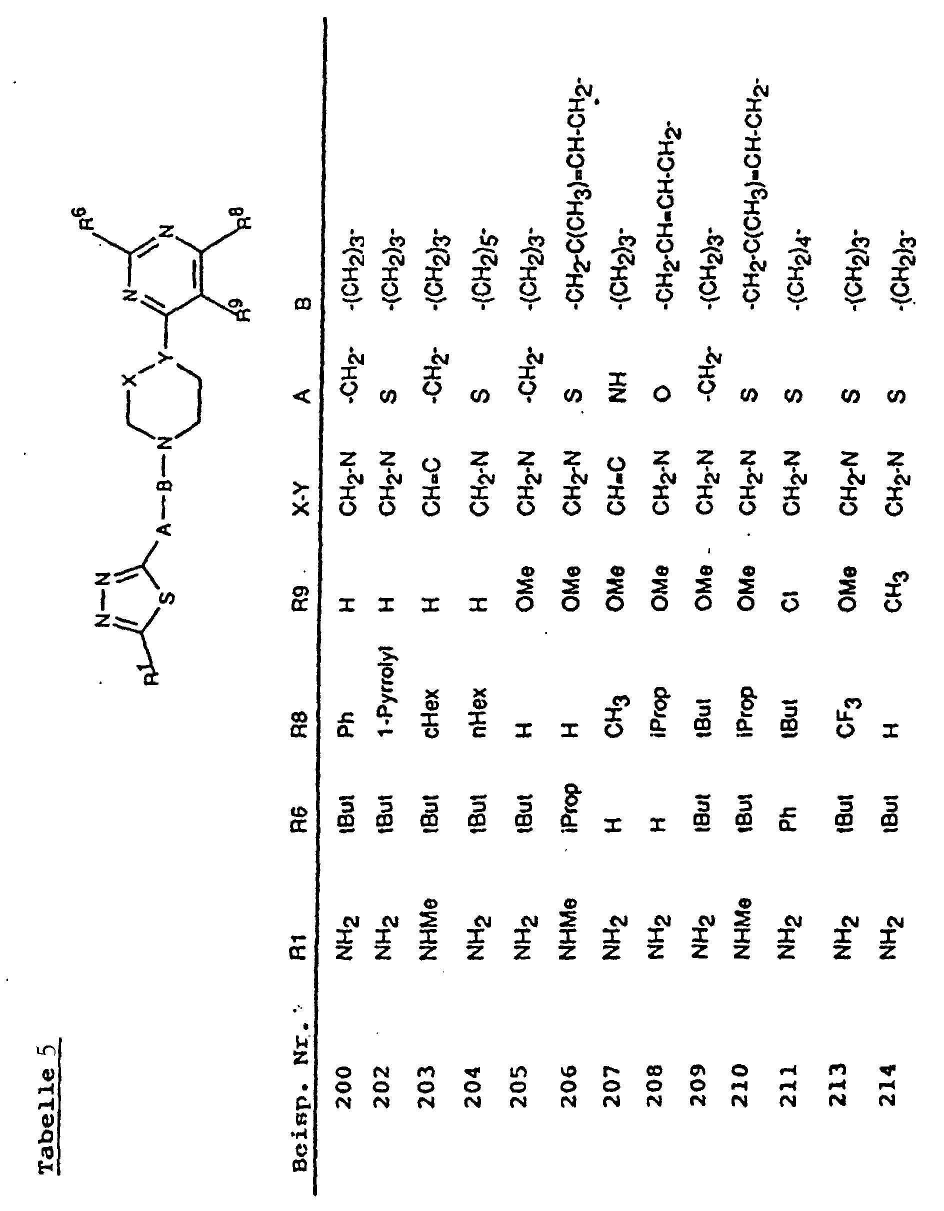

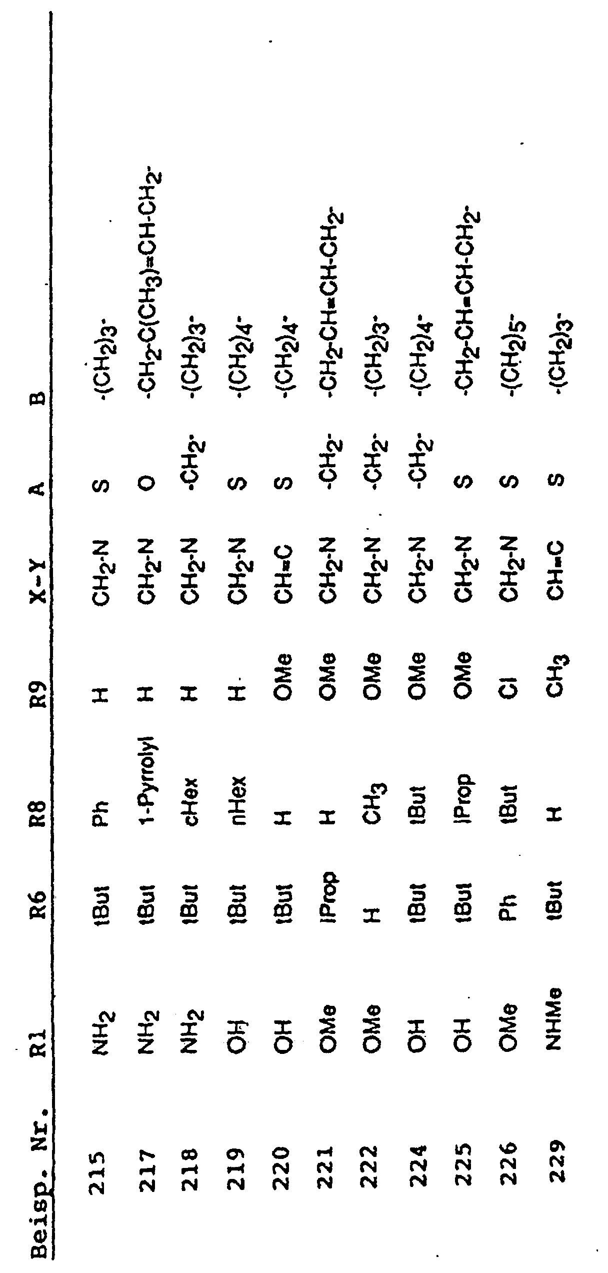

### Beispiele für galenische Applikationsformen:

### A) Tabletten

Auf einer Tablettenpresse werden in üblicher Weise Tabletten folgender Zusammensetzung gepreßt:

| | |
|---|---|
| 40 mg | Substanz des Referenz beispiel 1 |
| 120 mg | Maisstärke |
| 13,5 mg | Gelatine |
| 45 mg | Milchzucker |
| 2,25 mg | Aerosil® (chemisch reine Kieselsäure in submikroskopisch feiner Verteilung) |
| 6,75 mg | Kartoffelstärke (als 6 %iger Kleister) |

### B) Dragees

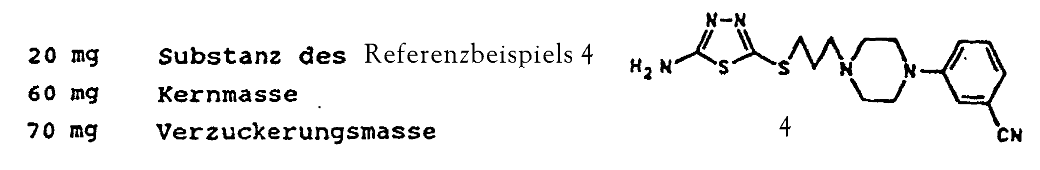

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus 5 Teilen Rohrzucker, Teilen Maisstärke, 2 Teilen Calciumcarbonat und 1 Teil Talk. Die so hergestellten Dragees werden anschließend mit einem magensaftresistenten Überzug versehen.

### Biologische Untersuchungen - Rezeptorbindungsstudien

### 1) D₃-Bindungstest

Für die Bindungsstudien wurden klonierte humane D₃-Rezeptor-exprimierende CCL 1,3 Mäusefibroblasten, erhältlich bei Res. Biochemicals Internat. One Strathmore Rd., Natick, MA 01760-2418 USA, eingesetzt.

### Zellpräparation

Die D₃ exprimierenden Zellen wurden in RPMI-1640 mit 10 % fötalem Kälberserum (GIBCO Nr. 041-32400 N); 100 E/ml Penicillin und 0,2 % Streptomycin (GIBCO BRL, Gaithersburg, MD, USA) vermehrt. Nach 48 h wurden die Zellen mit PBS gewaschen und mit 0,05 % trypsinhaltiger PBS 5 min inkubiert. Danach wurde mit Medium neutralisiert und die Zellen durch Zentrifugation bei 300 xg gesammelt. Zur Lyse der Zellen wurde kurz das Pellet mit Lysispuffer (5mM Tris-HCl, pH 7,4 mit 10 % Glycerin) gewaschen und danach in einer Konzentration von 10⁷-Zellen /ml Lysispuffer 30 min bei 4°C inkubiert. Die Zellen wurden bei 200 xg 10 min zentrifugiert und das Pellet in flüssigem Stickstoff gelagert.

### Bindungstests

Für den D₃-Rezeptorbindungstest wurden die Membranen in Inkubationspuffer (50 mM Tris-HCl, pH 7,4 mit 120 mM NaCl, 5 mM KCl, 2 mM CaCl₂, 2 mM MgCl₂, 10µM Quinolinol, 0,1 % Ascorbinsäure und 0,1 % BSA) in einer Konzentration von ca. 10⁶ Zellen/250 µl Testansatz suspendiert und bei 30°C mit 0,1 nM ¹²⁵Jodsulpirid in Anwesenheit und Abwesenheit von Testsubstanz inkubiert. Die unspezifische Bindung wurde mit 10⁻⁶M Spiperon bestimmt.

Nach 60 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 gewaschen. Die auf den Filtern gesammelte Radioaktivität wurde mit einem Packard 2200 CA Flüssigkeitszintillationszähler quantifiziert.

Die Bestimmung der Kᵢ-Werte erfolgte über nichtlineare Regressionsanalyse mit dem Programm LIGAND.

### 2) D₂-Bindungstest

### Membranpräparationen

a) Nucleus caudatus (Rind)
   Nucleus caudatus wurde aus Rinderhirn entfernt und in eiskalter 0,32 M Saccharose-Lösung gewaschen. Nach Gewichtsbestimmung wurde das Material zerkleinert und in 5 - 10 Volumen Saccharose-Lösung mit einem Potter-Evehjem Homogenisator (500 U/min) homogenisiert. Das Homogenat wurde bei 3000 x g 15 Minuten (4°C) zentrifugiert und der resultierende Überstand einer weiteren 15minütigen Zentrifugation bei 40000 x g unterworfen. Danach wurde der Rückstand zweimal mit 50 mM Tris-HCl, pH 7,4 durch Resuspension und Zentrifugation gewaschen. Die Membranen wurden bis zum Gebrauch in flüssigem N₂ gelagert.
b) Striatum (Ratte)
   Striati von Sprague-Dawley Ratten wurden in eiskalter 0,32 M Saccharose-Lösung gewaschen. Nach Gewichtsbestimmung wurden die Hirnteile in 5 - 10 Volumen Saccharose-Lösung mit einem Potter-Elvehjem Homogenisator (500 U/min) homogenisiert. Das Homogenat wurde bei 40000 x g 10 Minuten (4°C) zentrifugiert, danach wurde der Rückstand mit 50 mM Tris-HCl, 0,1 mM EDTA und 0,01 % Ascorbinsäure (pH 7,4) mehrmals durch Resuspension und Zentrifugation gewaschen. Der gewaschene Rückstand wurde mit dem obengenannten Puffer resuspendiert und 20 Minuten bei 37°C inkubiert (zwecks Abbau des endogenen Dopamins). Anschließend wurden die Membranen zweimal mit Puffer gewaschen und in Portionen in flüssigem Stickstoff eingefroren. Die Membranpräpration war maximal 1 Woche stabil.

### Bindungstest

a) ³H-Spiperon (D_{2low})
   Nucleus caudatus-Membranen wurden in Inkubationspuffer (mM: Tris-HCl 50, NaCl 120, KCl 5, MgCl₂ 1, CaCl₂ 2, pH 7,4) aufgenommen. Verschiedene Ansätze von je 1 ml wurden hergestellt:
   - Totale Bindung: 400 µg Membranen + 0,2 nmol/l 3H-Spiperon (Du Pont de Nemours, NET-565).
   - Unspezifische Bindung: wie Ansätze für totale Bindung + 10 µM (+)-Butaclamol.
   - Prüfsubstanz: wie Ansätze für totale Bindung + steigende Konzentrationen von Prüfsubstanz.

   Nach erfolgter Inkubation bei 25°C für 60 Minuten wurden die Ansätze über GF/B Glasfaserfilter (Whatman, England) an einem Skatron-Zellsammler (Fa. Zinsser, Frankfurt) filtriert und die Filter mit eiskaltem 50 mM Tris-HCl-Puffer, pH 7,4 gewaschen. Die auf den Filtern gesammelte Radioaktivität wurde mit einem Packard 2200 CA Flüssigkeitszintillationszähler quantifiziert.
   Die Bestimmung der Kᵢ-Werte erfolgte über nichtlineare Regressionsanalyse mit dem Programm LIGAND oder durch Umrechnung der IC₅₀-werte mit Hilfe der Formel von Cheng und Prusoff.
b) ³H-ADTN (D_{2high})
   Striatummembranen wurden in Inkubationspuffer (50 mM Tris-HCl, pH 7,4, 1 mM MnCl₂ und 0,1 % Ascorbinsäure) aufgenommen.
   Verschiedene Ansätze von je 1 ml wurden hergestellt.
   - Totale Bindung: 300 µg Naßgewicht + 1 nM ³H-ADTN (Du Pont de Nemours, Kundensynthese) + 100 nM SCH 23390 (Belegung von D1-Rezeptoren).
   - Unspezifische Bindung: wie Ansätze für totale Bindung + 50 nM Spiperon.
   - Prüfsubstanz: wie Ansätze für totale Bindung + steigende Konzentrationen von Prüfsubstanz.

   Nach erfolgter Inkubation bei 25°C für 60 Minuten wurden die Ansätze über GF/B Glasfaserfilter (Whatman, England) an einem Skatron-Zellsammler (Fa. Zinsser, Frankfurt) filtriert und die Filter mit eiskaltem 50 nM Tris-HCl-Puffer, pH 7,4 gewaschen. Die auf den Filtern gesammelte Radioaktivität wurde mit einem Packard 2200 CA Flüssigkeitszintillationszähler quantifiziert.
   Die Auswertung erfolgte wie unter a).

Die erfindungsgemäßen Verbindungen zeigen in diesen Tests sehr gute Affinitäten und hohe Selektivitäten gegenüber dem D₃-Rezeptor. Die erhaltenen Werte sind für repräsentative Verbindungen in der nachfolgenden Tabelle 6 zusammengestellt.

**Tabelle 6**

| Rezeptorbindung | | | |
|---|---|---|---|
| Beisp.Nr. | D₃ ¹²⁵ J-Sulpirid Kᵢ[nM] | D₂ ³H-Spiperon Kᵢ[nM] | Selektivität KᵢD₂/KᵢD₃ |
| 53 | 2,95 | 145 | 50 |
| 56 | 27,0 | 3500 | 70 |
| 58 | 1,7 | 225 | 132 |

## Patentansprüche

1. Thiadiazolverbindungen der Formel I, 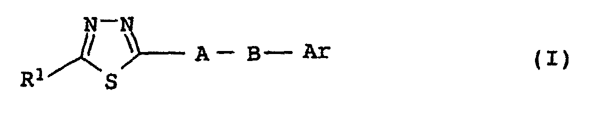 worin
A für eine geradkettige oder verzweigte C₃-C₁₄-Alkylengruppe steht, die gegebenenfalls wenigstens eine Gruppe umfasst, die ausgewählt ist unter O, S, NR³ und einer Doppel- oder Dreifachbindung,
B für einen Rest der Formel steht: 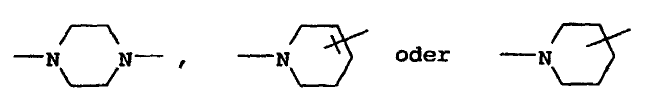
R¹ für Wasserstoff, OR³, NR²R³ oder für C₁-C₈-Alkyl steht,
R² und R³ unabhängig voneinander für H oder C₁-C₈-Alkyl stehen,
Ar für Pyridinyl, Pyrimidinyl oder Triazinyl steht, wobei Ar ein bis vier Substituenten aufweist, die ausgewählt sind unter OR³, C₁-C₈-Alkyl, C₂-C₈-Alkinyl, C₂-C₈-Alkenyl, Halogen, CN, CO₂R², NO₂, SO₂R², SO₃R², NR²R³, SO₂NR²R³, SR², CHF₂, CF₃, einem 5- oder 6-gliedrigen, carbocyclischen, aromatischen oder nicht aromatischen Ring und einem 5- oder 6-gliedrigen, heterocyclischen, aromatischen oder nicht aromatischen Ring mit 1 bis 3 Heteroatomen, die ausgewählt sind unter 0, S und N, wobei der carbocyclische oder der heterocyclische Ring gegebenenfalls substituiert ist durch C₁-C₈-Alkyl, OC₁-C₈-Alkyl, Halogen, OH, NO₂, oder CF₃, oder worin Ar mit einem carbocyclischen oder heterocyclischen Ring der oben genannten Art kondensiert ist,
sowie deren Salze mit physiologisch verträglichen Säuren.

2. Thiadiazolverbindungen nach Anspruch 1, worin Ar in Formel I für Pyridinyl oder Pyrimidinyl steht, das einen, zwei, drei oder vier Substituenten aufweist, die ausgewählt sind unter C₁-C₈-Alkyl, Phenyl, C₅-C₆-Cycloalkyl, einem 5- oder 6-gliedrigen, heterocyclischen, aromatischen oder nicht aromatischen Rest mit 1 bis 3 Heteroatomen, die ausgewählt sind unter O, S und N, CHF₂, CF₃, Halogen, CN, NO₂, OR³ und SR², wobei R² und R³ die oben angegebenen Bedeutungen besitzen.

3. Thiadiazolverbindungen nach Anspruch 2, worin B in Formel I für: 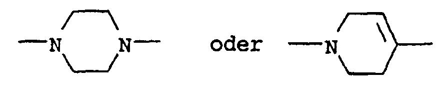 steht.

4. Thiadiazolverbindungen nach Anspruch 1, worin Ar in Formel I für Pyrimidinyl steht, das ein bis drei Substituenten aufweist, die unabhängig voneinander ausgewählt sind unter C₁-C₈-Alkyl, C₅-C₆-Cycloalkyl, Phenyl, Pyrrolyl, OH, OC₁-C₈-Alkyl, CHF₂, CF₃ und Halogen.

5. Thiadiazolverbindungen nach Anspruch 1, worin Ar in Formel I für Pyridinyl steht, das ein bis vier Substituenten aufweist, die unabhängig voneinander ausgewählt sind unter C₁-C₈-Alkyl, C₂-C₈-Alkenyl, C₂-C₈-Alkinyl, Phenyl, Pyrrolyl, OH, OC₁-C₈-Alkyl, CHF₂, CF₃, CN und Halogen.

6. Verwendung von Thiadiazolverbindungen der Formel I gemäß einem der vorhergehenden Ansprüchen sowie der Salze davon mit physiologisch verträglichen Säuren zur Herstellung eines pharmazeutischen Mittels zur Behandlung von Erkrankungen, die auf Dopamin-D3-rezeptorantagonisten oder -agonisten ansprechen.

7. Pharmazeutisches Mittel, enthaltend mindestens eine Thiadiazolverbindung der Formel I gemäß einem der vorhergehenden Ansprüche 1 bis 5 oder ein Salz davon mit physiologisch verträglichen Säuren, gegebenenfalls zusammen mit physiologisch akzeptablen Trägern und/oder Hilfsstoffen.

## Claims

1. Thiadiazole compounds of the formula I: 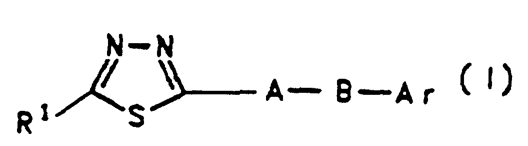 where
A is a straight-chain or branched C₃-C₁₄-alkylene group which optionally comprises at least one group selected from among O, S, NR³ and a double or triple bond,
B is a radical of the formula: 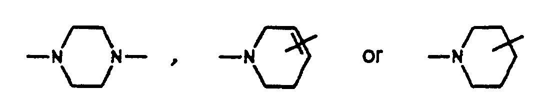
R¹ is hyrogen, OR³, NR²R³, or C₁-C₈-alkyl
R² and R³ are, independently of one another, H or C₁-C₈-alkyl;
Ar is pyridinyl, pyrimidinyl or triazinyl, where Ar may have from one to four substituents which are selected from OR³, C₁-C₈-alkyl, C₂-C₈-alkynyl, C₂-C₈-alkenyl, halogen, CN, CO₂R², NO₂, SO₂R², SO₃R², NR²R³, SO₂NR²R³, SR², CHF₂, CF₃, a 5- or 6-membered carbocyclic aromatic or non-aromatic ring and a 5- or 6- membered heterocyclic aromatic or non-aromatic ring having 1 to 3 heteroatoms selected from O, S and N, where the carbocyclic or the heterocyclic ring is optionally substituted by C₁-C₈-alkyl, OC₁-C₈-alkyl, halogen OH, NO₂ or CF₃ or where Ar may also be fused to a carbocyclic or heterocyclic ring of the type defined above,
and the salts thereof with physiologically tolerated acids

2. Thiadiazole compound as claimed in claim 1 where Ar in formula I is pyridinyl or pyrimidinyl which has one, two, three or four substituents which are selected from C₁-C₈-alkyl, phenyl, OR³, C₅-C₆-cycloalkyl, a 5- or 6-membered heterocyclic aromatic or non-aromatic radical having 1 to 3 heteroatoms selected from O, S and N CHF₂, CF₃, halogen, CN, NO₂ and SR², R² and R³ having the meanings given above.

3. Thiadiazole compounds as claimed in claim 2, where B in formula I is

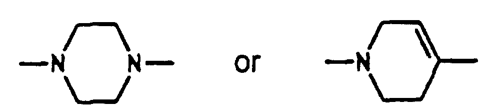

4. Thiadiazole compounds as claimed in claim 1, where Ar in formula I is pyrimidinyl which has one to three substituents which are selected, independently of one another, from C₁-C₈-alkyl, C₅-C₆-cycloalkyl, phenyl, pyrrolyl, OH, OC₁-C₈-alkyl, CHF₂, CF₃ and halogen.

5. Thiadiazole compounds as claimed in claim 1, where Ar in formula I is pyridinyl which has one to four substituents which are selected, independently of one another, from C₁-C₈-alkyl, C₂-C₈-alkenyl, C₂-C₈-alkynyl, phenyl, pyrrolyl, OH, OC₁-C₈-alkyl, CHF₂, CF₃, CN and halogen.

6. The use of thiadiazole compounds of the formula I as claimed in any of the preceding claims, and of the salts thereof with physiologically tolerated acids for preparing a pharmeuctical composition for treating disorders which respond to dopamine D3 receptor antagonists or agonists.

7. A pharmaceutical composition which comprises at least one thiadiazole compound of the formula I as claimed in any of the preceding claims 1 to 5 or a salt thereof with physiologically tolerated acids, optionally together with physiologically acceptable carriers and/or excipients.

## Revendications

1. Composés de thiadiazole de formule I, 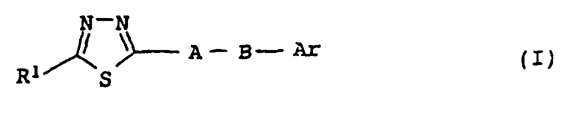 où
A désigne un groupe alkylène en C₃-C₁₄ linéaire ou ramifié, qui comporte éventuellement au moins un groupe qui est choisi parmi O, S, NR³ et une liaison double ou triple,
B représente un reste de formule: 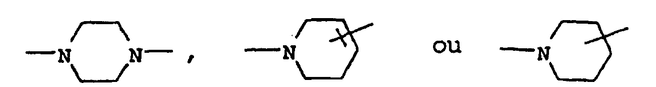
R¹ désigne l'hydrogène, OR³, NR²R³ ou un alkyle en C₁-C₈,
R² et R³ représentent indépendamment l'un de l'autre H ou un alkyle en C₁-C₈,
Ar représente un pyridinyle, pyrimidinyle ou triazinyle, tandis que Ar présente un à quatre substituants qui sont choisis parmi OR³, alkyle en C₁-C₈, alcynyle en C₂-C₈, alcényle en C₂-C₈, halogène, CN, CO₂R², NO₂, SO₂R², SO₃R², NR²R³, SO₂NR²R³, SR², CHF₂, CF₃, un cycle carbocyclique, aromatique ou non-aromatique, ayant 5 ou 6 chaînons, et un cycle hétérocyclique, aromatique ou non-aromatique, ayant 5 ou 6 chaînons, comportant 1 à 3 hétéroatomes, qui sont choisis parmi O, S et N, tandis que le cycle carbocyclique ou le cycle hétérocyclique est éventuellement substitué par des groupes alkyle en C₁-C₈, O-alkyle en C₁-C₈, halogène, OH, NO₂, ou CF₃, ou dans lequel Ar est condensé avec un cycle carbocyclique ou hétérocyclique du type indiqué plus haut,
ainsi que leurs sels avec des acides physiologiquement acceptables.

2. Composés de thiadiazole selon la revendication 1, dans lesquels Ar dans la formule I désigne un pyridinyle ou un pyrimidinyle, qui présente un, deux, trois ou quatre substituants qui sont choisis parmi les groupes alkyle en C₁-C₈, phényle, cycloalkyle en C₅-C₆, un reste hétérocyclique, aromatique ou non-aromatique, ayant 5 ou 6 chaînons, comportant 1 à 3 hétéroatomes qui sont choisis parmi O, S et N, CHF₂, CF₃, halogène, CN, NO₂, OR³ et SR₂, tandis que R² et R³ possèdent les significations indiquées plus haut.

3. Composés de thiadiazole selon la revendication 2, où B dans la formule I désigne:

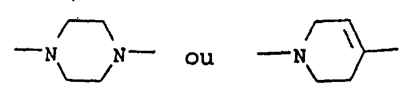

4. Composés de thiadiazole selon la revendication 1, dans lesquels Ar dans la formule I désigne un pyrimidinyle qui présente un à trois substituants qui sont choisis, indépendamment l'un de l'autre, parmi les groupes alkyle en C₁-C₈, cycloalkyle en C₅-C₆, phényle, pyrrolyle, OH, O-alkyle en C₁-C₈, CHF₂, CF₃ et halogène.

5. Composés de thiadiazole selon la revendication 1, dans lesquels Ar dans la formule I désigne un groupe pyridinyle qui présente un à quatre substituants qui sont choisis, indépendamment l'un de l'autre, parmi les groupes alkyle en C₁-C₈, alcényle en C₂-C₈, alcynyle en C₂-C₈, phényle, pyrrolyle, OH, O-alkyle en C₁-C₈, CHF₂, CF₃, CN et halogène.

6. Utilisation de composés de thiadiazole de formule I selon l'une des revendications précédentes, ainsi que de leurs sels avec des acides physiologiquement acceptables, pour la. préparation d'un produit pharmaceutique pour le traitement d'affections qui réclament des antagonistes ou des agonistes du récepteur de la dopamine D3.

7. Produit pharmaceutique, contenant au moins un composé de thiadiazole de formule I selon l'une des revendications précédentes 1 à 5, ou un sel de celui-ci avec des acides physiologiquement acceptables, éventuellement conjointement avec des supports et/ou des adjuvants physiologiquement acceptables.
